# EUROPEAN PATENT APPLICATION

(11) **EP 4 516 333 A1**
(43) Date of publication of application: **05.03.2025**
(21) Application number: 23796299.8
(22) Date of filing: 24.04.2023
(51) Int. Cl.: A61M 1/36, A61M 1/16

(54) **CONNECTION DEVICE**

(30) Priority: 25.04.2022 JP 2022071957
(71) Applicant: Keio University, Tokyo, 108-8345 (JP)
(72) Inventor: MIKI, Norihisa, Yokohama-shi, Kanagawa 223-8522 (JP); OTA, Takashi, Yokohama-shi, Kanagawa 223-8522 (JP); KONO, Rei, Yokohama-shi, Kanagawa 223-8522 (JP)
(74) Representative: Peters, Sebastian Martinus
(86) International application number: PCT/JP2023/016044
(87) International publication number: WO 2023/210559

(57) **Abstract**

Provided are a connection device in which flow stagnation does not readily occur, and a method for manufacturing the same. This connection device (1) has an internal cavity (9) for connecting, in a state allowing fluid transport, a flow path (3) and a housing (7) accommodating membranes (5), the connection device (1) also having a plurality of flow path adjustment walls (13) provided in the internal cavity (9). The method for manufacturing the connection device (1) includes a step for installing, in a main body section (11) of the connection device (1), a plurality of virtual flow path layers having thicknesses corresponding to inter-membrane flow paths (17) formed by a plurality of stacked membranes (5), a step for forming the plurality of flow path adjustment walls (13) among the plurality of virtual flow path layers, and a step for obtaining inter-adjustment-wall flow paths (15) formed by the plurality of flow path adjustment walls (13) by forming the plurality of flow path adjustment walls (13) and then removing the virtual flow path layers.

## Description

### TECHNICAL FIELD

The present invention relates to a connection device for connecting a flow channel and a film. The present invention relates, for example, to a connection device used for connecting a blood flow channel and the main body of a medical instrument including a film.

### BACKGROUND ART

Japanese Patent Publication No. 2017-158814 describes a blood filtration device. The main body section of this device includes a plurality of blood flow channel layers, a filtration film and a filtrate flow channel layer. Each blood inflow section of each blood flow channel layer of this device is connected to a single blood inlet. Thus, when blood flows in this device, there are portions where the direction of the blood flow changes, or the diameter varies. In this device, blood also deposits on the end surfaces of stacked materials. As a result, blood retention occurs in this device, leading to clot formation.

### CITATION LIST

### PATENT LITERATURE

Patent Literature 1: Japanese Patent Publication No. 2017-158814.

### SUMMARY OF THE INVENTION

### TECHNICAL PROBLEM

The objective of the present invention is to provide a connection device in which fluid flow stagnation does not readily occur when connecting a flow channel and a housing accommodating a film.

Specifically, the objective of the present invention is to provide a connection device that can reduce the occurrence of clots when connecting a blood flow channel and a medical instrument including a film.

### SOLUTION TO THE PROBLEM

The above problem is basically based on the finding that it can be solved by providing a plurality of flow channel adjustment walls in an internal cavity of a connection device to adjust a fluid flow channel.

The first invention described in the present description relates to the connection device 1. The connection device 1 is a device for connecting a flow channel 3 and a housing 7 in a state allowing fluid transport. The connection device 1 includes the plurality of flow channel adjustment walls 13 provided in the internal cavity 9. A fluid from the flow channel 3 is carried through the internal cavity 9 of the connection device 1 into the housing 7. The housing 7 accommodates a film 5. Thus, the fluid from the flow channel 3 is carried through the internal cavity 9 of the connection device 1 to the film 5. When this occurs, the flow of the fluid is adjusted by the plurality of flow channel adjustment walls 13.

The next invention described in the present description relates to a method for manufacturing the connection device 1 described above.

The method includes a step of installing a virtual flow channel layer, a step of forming the flow channel adjustment wall and a step of obtaining an inter-adjustment-wall flow channel.

The step of installing the virtual flow channel layer is a step of installing a plurality of virtual flow channel layers 23 having the thickness corresponding to an inter-film flow channel 17 formed by a plurality of stacked films 5 in a main body section 11 of the connection device 1.

The step of forming the flow channel adjustment wall is a step of forming the plurality of flow channel adjustment walls 13 between the plurality of virtual flow channel layers 23.

The step of obtaining the inter-adjustment-wall flow channel is a step of obtaining the inter-adjustment-wall flow channel 15 formed by the plurality of flow channel adjustment walls 13 by forming the plurality of flow channel adjustment walls 13 and then removing the virtual flow channel layers 23.

### ADVANTAGEOUS EFFECTS OF THE INVENTION

In the present invention, the plurality of flow channel adjustment walls are provided to adjust the fluid flow channel. This prevents fluid flow stagnation when connecting the flow channel and the housing accommodating the film. Since this connection device includes the plurality of flow channel adjustment walls, it can reduce the occurrence of clots when connecting the blood flow channel and the film.

### BRIEF DESCRIPTION OF THE DRAWINGS

FIG. 1 is a conceptual diagram of a connection device.
FIG. 2 is a conceptual diagram illustrating how the connection device, a flow channel and a housing are connected.
FIG. 3 is a conceptual diagram illustrating how a fluid from the flow channel is carried through an internal cavity of the connection device to a film inside the housing. FIG. 3(a) is a diagram for describing a prior art, and FIG. 3(b) is a diagram for describing the present invention.
FIG. 4 is a conceptual diagram for describing a step of installing a virtual flow channel layer. FIG. 4(a) is a diagram illustrating how a plurality of sheets are installed. FIG. 4(b) is a diagram illustrating how the plurality of sheets are grouped together. FIG. 4(c) is a diagram illustrating how a cover is attached.
FIG. 5 is a conceptual diagram for describing a step of forming a flow channel adjustment wall. FIG. 5(a) is a diagram illustrating how a resin is put inside the cover. FIG. 5(b) is a diagram illustrating how the resin is cured.
FIG. 6 is a diagram illustrating a step of obtaining an inter-adjustment-wall flow channel. FIG. 6(a) is a conceptual diagram illustrating how the virtual flow channel layer is removed. FIG. 6(b) is a diagram illustrating how the cover is removed to obtain the connection device.
FIG. 7 is an exploded diagram of a blood filtration device.
FIG. 8 is a photograph in lieu of a drawing illustrating the connection device obtained in an example.
FIG. 9 is a photograph in lieu of a drawing illustrating an example of the use of the connection device. FIG. 9(a) illustrates what the connection device without the flow channel adjustment wall looks like after using it. FIG. 9(b) illustrates an example of using the connection device provided with the flow channel adjustment wall.

### DESCRIPTION OF EMBODIMENTS

An embodiment of the present invention will be explained below with reference to the drawings. The present invention is not limited to the embodiment described below and incorporates modifications from the embodiment below to the extent that they are obvious to those skilled in the art.

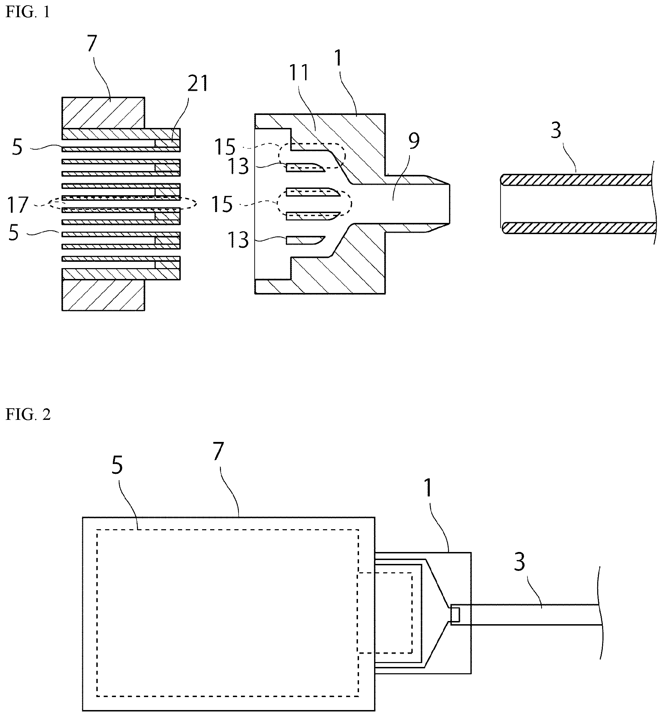

FIG. 1 is a conceptual diagram of a connection device. As shown in FIG. 1, the connection device 1 includes a main body section 11, an internal cavity 9, which is a space inside the main body section 11, and a plurality of flow channel adjustment walls 13 provided in the inner cavity 9. As shown in FIG. 1, a space between two adjacent walls out of the plurality of flow channel adjustment walls 13 present in the internal cavity 9 forms an inter-adjustment-wall flow channel 15. A space between a wall surface of the internal cavity 9 and an outermost one of the flow channel adjustment walls 13 may also form the inter-adjustment-wall flow channel 15. FIG. 1 depicts not only the connection device 1 but also a flow channel 3 and a housing 7. FIG. 2 is a conceptual diagram illustrating how the connection device, the flow channel and the housing are connected.

The connection device 1 is a device for connecting the flow channel 3 and the housing 7 in a state allowing fluid transport. The connection device 1 is a device intended to transport a fluid from the flow channel 3 to a film 5 in the housing 7 with reduced stagnation of the fluid. Therefore, the connection device 1 can be preferably used when the inner diameter of the end portion of the flow channel 3 differs from the inner diameter of the end portion of the housing 7. The connection device 1 can be used to connect a blood flow channel and a portion accommodating the film in, for example, an implantable artificial kidney, a portable artificial kidney, a hemodialysis machine (dialyzer), a blood filtration device (hemodiafilter), an extracorporeal film oxygenator (ECMO) or a continuous hemodiafiltration device. The connection device 1 can be preferably used in a medical instrument for carrying blood from a blood flow channel to the film.

The connection device 1 can be molded using a publicly known material. Examples of the material for the connection device 1 include polyvinyl chloride, polyethylene, polypropylene, cyclic polyolefin, polystyrene, polycarbonate, acrylic resin, methacrylic resin, acrylonitrile-butadiene-styrene copolymer, polyethylene terephthalate, butadiene-styrene copolymer, polyamide (for example, nylon 6, nylon 6.6, nylon 6.10, nylon 12), natural rubber, butyl rubber, isoprene rubber, butadiene rubber, styrene-butadiene rubber, silicone rubber, various thermoplastic elastomers, PTFE (polytetrafluoroethylene), ePTFE (expanded polytetrafluoroethylene), titanium, stainless steel, aluminum, copper and copper alloys.

The connection device 1 can be shaped to fit the flow channel 3 and the housing 7. The shapes of the connection device 1 as well as the internal cavity 9 therein and the main body section 11 may also be appropriate for the intended use.

### Flow channel 3

The flow channel 3 is a pathway for the fluid to carry the fluid in the flow channel 3 to the film in the housing 7 by being connected to the housing 7 via the connection device 1. Examples of the fluid that flows through the flow channel include blood, dialysate, condensate liquid, saline solution, perfusion fluid, drip infusion fluid and water. Among these, blood is preferred. An example of the inner diameter of the flow channel 3 is 2 mm or more and 2 cm or less. The inner diameter of the flow channel may be 2 mm or more and 8 mm or less, 3 mm or more and 7 mm or less, 3 mm or more and 5 mm or less, or 2 mm or more and 4 mm or less. Examples of the flow channel 3 include an artificial blood vessel and a tube.

### Housing 7

The housing 7 is an element for accommodating the film 5 therein. The housing may be the main body of the medical instrument that includes the film therein. An example of the housing is the hexagonal column-shaped main body section of the blood filtration device (Japanese Patent Publication No. 2017-158814). The housing 7 functions, for example, as a filtration device since it has the film 5 therein. For example, the film 5 may be a planar film or a hollow-fiber film. If the film 5 is planar, it may be a stacked structure of a plurality of films. In the case where the film 5 inside the housing 7 is the stacked structure of the plurality of films, a connector 21 may exist between the films. In the case where it is the stacked structure of the plurality of films, an inter-film flow channel 17 exists between the films through which the fluid to be filtered, etc., flows. An example of the film 5 is a hemodiafiltration film. The pore size of the hemodiafiltration film is preferably 0.1 nm or more and 10 nm or less, and may be 0.5 nm or more and 2 nm or less. This size prevents viruses and bacteria from passing through, preventing any viruses or bacteria from mixing with blood and thus preventing infections.

### Internal cavity 9

The internal cavity 9 refers to a space (area) inside the main body section 11 of the connection device 1. The main body section 11 of the connection device 1 is an enclosure (outer box) including the internal cavity 9 therein. The connection device 1 is a device for connecting the flow channel 3 and the housing 7 in a state allowing fluid transport. For example, the area of the cross-section of the internal cavity (area through which the fluid passes) of the end portion of the flow channel 3 that is connected to the connection device 1 may be smaller than the area of the internal cavity (area through which the fluid passes) of the end portion of the housing 7 that is connected to the connection device 1. Thus, the flow channel 3 cannot be directly connected to the housing 7. The internal cavity 9 of the connection device 1 includes a first end fitted to the shape of the end portion of the flow channel 3 and a second end fitted to the end portion of the housing 7. Here, "fitted" means that the physical connection is secured so that the fluid can be carried without any leakage or stagnation. Therefore, the connection device 1 helps the flow channel 3 to be connected to the housing 7, and the smooth shape of the internal cavity 9 reduces fluid flow stagnation. An example of the shape of the internal cavity 9 is one that includes an introduction portion leading from the first end and fitted to the shape of the internal cavity of the end portion of the flow channel 3, an exit portion leading to the second end and fitted to the shape of the internal cavity of the end portion of the housing 7, and a coupling portion smoothly coupling the introduction portion and the exit portion.

As described above, if the area of the cross-section of the internal cavity of the end portion of the housing 7 is larger than the area of the cross-section of the internal cavity of the end portion of the flow channel 3, the internal cavity 9 preferably includes a portion whose cross-sectional area increases in the direction from the end portion of the flow channel 3 to the end portion of the housing side. The outer wall portion of the internal cavity 9 preferably has a shape whose cross-sectional area gradually increases in the direction from the end portion of the flow channel 3 to the end portion of the housing side. In particular, the cross-section of the internal cavity portion of the flow channel 3 is generally circular (including elliptical) to allow the fluid to flow without stagnation. Meanwhile, if the film 5 inside the housing 7 is the stacked structure of the plurality of films, the shape of the internal cavity portion of the end portion of the housing 7 may be rectangular (square or quadrilateral). As such, if the shape of the internal cavity portion of the end portion of the flow channel 3 is different from the shape of the internal cavity portion of the end portion of the housing 7, fluid flow stagnation may occur when connecting the flow channel 3 and the housing 7. This connection device 1 can make the fluid flow smooth. For example, when the fluid is blood, stagnation of the fluid may lead to clot formation. This connection device 1 makes the flow of blood smooth and effectively reduces the occurrence of clots.

### Flow channel adjustment wall 13

The flow channel adjustment wall 13 refers to the plurality of walls provided in the internal cavity 9. The inter-adjustment-wall flow channel 15, the area through which the fluid flows, is formed between the two adjacent flow channel adjustment walls 13. The inter-adjustment-wall flow channel 15 may also be formed between the outer wall of the internal cavity 9 and the outermost one of the flow channel adjustment walls 13. When the shape of the internal cavity 9 changes in the direction from the first end to the second end, the flow velocity of the fluid that flows through the internal cavity 9 changes. This change is preferably minimized as much as possible. The flow channel adjustment wall 13 reduces the change in the area of the cross-section of the flow channel. In addition, the flow channel adjustment walls 13 form the inter-adjustment-wall flow channel 15, thereby making the flow of the fluid smooth.

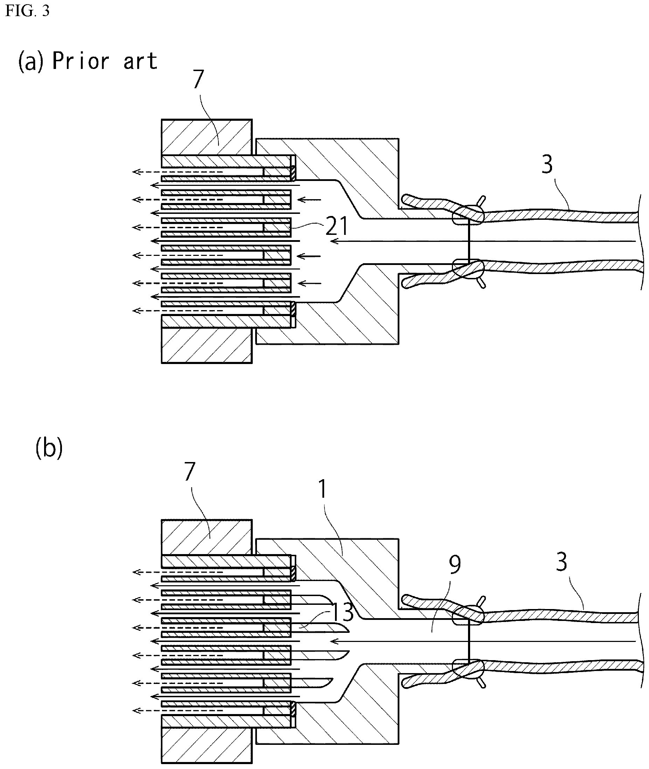

FIG. 3 is a conceptual diagram illustrating how the fluid from the flow channel is carried through the internal cavity of the connection device to the film inside the housing. FIG. 3(a) is a diagram for describing a prior art, and FIG. 3(b) is a diagram for describing the present invention. When the film 5 inside the housing 7 is the stacked structure of the plurality of films, the fluid colliding the connector 21 causes fluid flow stagnation. For example, when the fluid is blood, a clot may form at the site of collision with this connector. For this reason, a preferred example of the flow channel adjustment wall 13 is to form the flow channel adjustment wall 13 so that it is contiguous with this connector 21. Then, since the inter-adjustment-wall flow channel 15 and the inter-film flow channel 17 are coupled, the fluid that flows through the internal cavity 9 is smoothly carried through the plurality of stacked films 5. Note that when the film 5 is formed on both side surfaces of the connector 21 as shown in FIG. 3(b), the flow channel adjustment wall 13 may be formed to be contiguous with the connector 21 and the film 5. This configuration makes the flow of the fluid smooth, even when the thickness of the film 5 is not negligible. Note that the figure illustrates how the fluid from the flow channel is carried through the internal cavity of the connection device to the film inside the housing. Meanwhile, this connection device can also be preferably used when the fluid is carried from the inside of the housing to a flow channel for fluid discharge. However, the flow velocity and the state of the fluid when the fluid from the flow channel is carried through the internal cavity of the connection device to the film inside the housing is different from that when the fluid from the housing is carried through the connection device to the flow channel for fluid discharge. Therefore, the inter-adjustment-wall flow channel 15 is preferably designed with those conditions taken into consideration.

When the internal cavity 9 includes the introduction portion, the coupling portion and the exit portion, the flow channel adjustment wall 13 is preferably provided in the coupling portion and the exit portion, or in the exit portion. This configuration can reduce the risk of sudden changes in the area of the cross-section through which the fluid flows. The plurality of flow channel adjustment walls 13 are preferably provided so that they are parallel. When the plurality of flow channel adjustment walls 13 are provided in parallel, the inter-adjustment-wall flow channel 15 includes a straight portion, facilitating the flow of the fluid.

The flow channel adjustment wall 13 may be formed by convex portions that are periodically provided, or a riblet structure may be employed.

The next invention described in the present description relates to a method for manufacturing the connection device 1 described above.

The method includes a step of installing a virtual flow channel layer, a step of forming the flow channel adjustment wall and a step of obtaining the inter-adjustment-wall flow channel.

### Step of installing the virtual flow channel layer

The step of installing the virtual flow channel layer is a step of installing a plurality of virtual flow channel layers 23 having the thickness corresponding to the inter-film flow channel 17 formed by the plurality of stacked films 5 in the main body section 11 of the connection device 1. The main body section 11 of the connection device 1 is a housing (enclosure, outer box) including the internal cavity 9 therein.

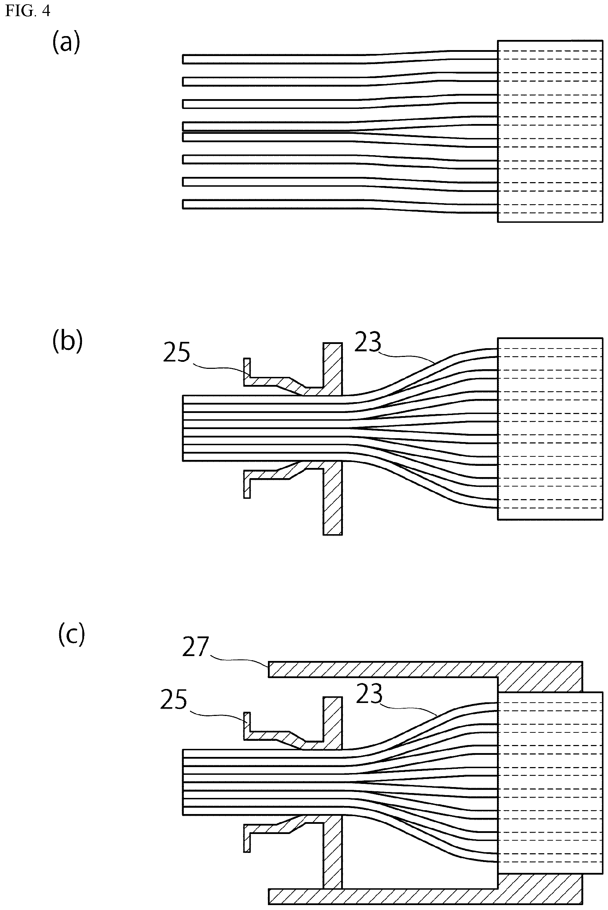

FIG. 4 is a conceptual diagram for describing the step of installing the virtual flow channel layer. FIG. 4(a) is a diagram illustrating how a plurality of sheets are installed. FIG. 4(b) is a diagram illustrating how the plurality of sheets are grouped together. FIG. 4(c) is a diagram illustrating how a cover is attached. As shown in FIG. 4(a), the plurality of sheets are installed at a position that corresponds to the space between the connectors 21. Note that when the housing and the connection device 1 are molded as one piece, the sheet is installed in the inter-film flow channel 17, which is located between the connectors 21. This sheet later serves as the inter-adjustment-wall flow channel 15. Thus, the size and thickness of the sheet may be adjusted to match the inter-film flow channel 17 and the inter-adjustment-wall flow channel 15. Next, as shown in FIG. 4(b), the plurality of sheets are grouped together by a connection portion 25. This connection portion 25 is an element that is to be part of the connection device 1 and may be a joint portion with the flow channel 3. Next, as shown in FIG. 4(c), the cover to form the area that corresponds to the main body section of the connection device is attached. In this way, the virtual flow channel layer 23 is formed in the area that is to be the connection device.

### Step of forming the flow channel adjustment wall

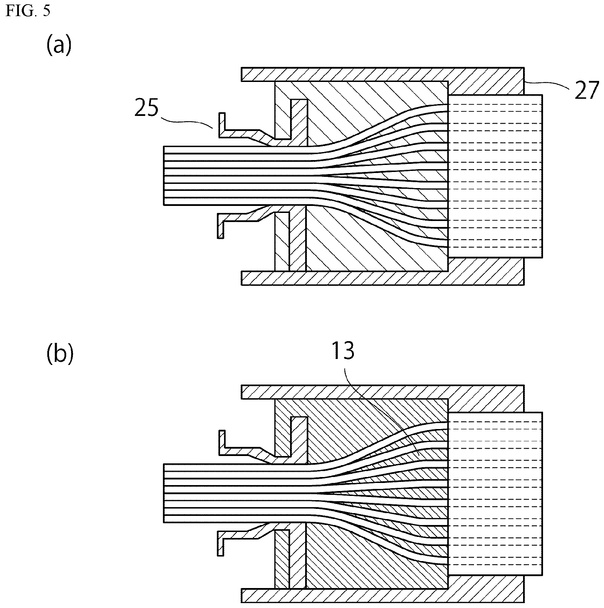

The step of forming the flow channel adjustment wall is a step of forming the plurality of flow channel adjustment walls 13 between the plurality of virtual flow channel layers 23. FIG. 5 is a conceptual diagram for describing the step of forming the flow channel adjustment wall. FIG. 5(a) is a diagram illustrating how a resin is put inside the cover. FIG. 5(b) is a diagram illustrating how the resin is cured. As shown in FIG. 5(a), the resin is put inside the cover. Then, as shown in FIG. 5(b), the resin is cured. Then, the plurality of flow channel adjustment walls 13 are formed between the plurality of virtual flow channel layers 23. Such a resin is publicly known. When forming the flow channel adjustment wall 13 by this method, a thermosetting resin or a photocurable resin can be preferably used. Thermosetting resins and photocurable resins are publicly known. An example of the thermosetting resin is an epoxy compound containing an aromatic group that is thermally stable at high temperatures. The photocurable resin may be obtained by adding a photo-acid-generating agent to a thermosetting resin. Examples of the photo-acid-generating agent are SbF₆⁻, PF₆⁻, BF₄⁻ and AsF₆⁻. The content of the photo-acid-generating agent ranges from 0.5 to 2.0 parts by mass with respect to 100 parts by mass of the thermosetting resin. When using a photocurable resin, the cover preferably has a property that allows light to pass through, for example, by making the cover transparent.

### Step of obtaining the inter-adjustment-wall flow channel

The step of obtaining the inter-adjustment-wall flow channel is a step of obtaining the inter-adjustment-wall flow channel 15 formed by the plurality of flow channel adjustment walls 13 by forming the plurality of flow channel adjustment walls 13 and then removing the virtual flow channel layers 23.

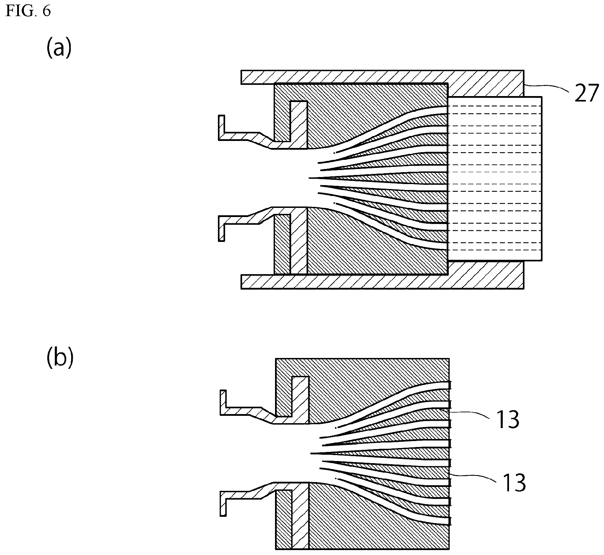

FIG. 6 is a diagram illustrating the step of obtaining the inter-adjustment-wall flow channel. FIG. 6(a) is a conceptual diagram illustrating how the virtual flow channel layer is removed. FIG. 6(b) is a diagram illustrating how the cover is removed to obtain the connection device. The virtual flow channel layer 23 as well as the cover is removed. When the virtual flow channel layer 23 is removed, the inter-adjustment-wall flow channel 15 is formed at that position. FIG. 6(a) is a conceptual diagram illustrating how the virtual flow channel layer is removed. Note that the cover may be removed before removing the virtual flow channel layer. As shown in FIG. 6(b), when the connection device 1 is not molded as one piece with the housing 7, the portion that corresponds to the film 5 may be removed. In this way, the connection device can be obtained. The connection device is preferably molded as one piece with the housing 7 and the film 5 therein. By molding the connection device and the housing 7 as well as the film 5 therein as one piece, interfaces between components that normally require the use of packing, etc., to prevent leaks are eliminated. The above description focuses on the case where the film 5 is a stacked type. In contrast, when the film 5 is a hollow-fiber type, the connection device can be manufactured using a rod-shaped body with the same diameter as the hollow fiber, in the same way as when the film 5 is a stacked type.

The above describes a method for manufacturing the connection device using the housing 7 that includes the plurality of stacked films 5. Meanwhile, the connection device may be manufactured together with the housing 7 when manufacturing it.

The fluid from the flow channel 3 is carried through the internal cavity 9 of the connection device 1 into the housing 7. The connection device 1 includes the plurality of flow channel adjustment walls 13 provided in the internal cavity 9. The housing 7 accommodates the film 5. Thus, the fluid from the flow channel 3 is carried through the inter-adjustment-wall flow channel 15 formed by the flow channel adjustment wall 13 present in the internal cavity 9 of the connection device 1 to the film 5 in the housing 7.

### Blood filtration device

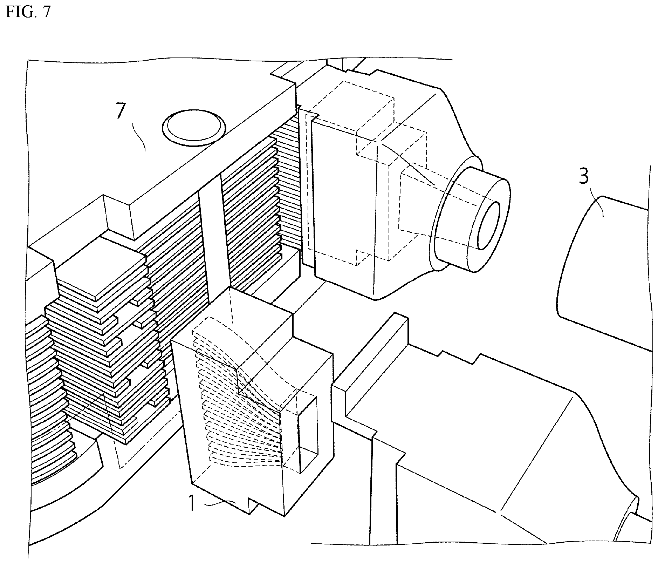

FIG. 7 is an exploded diagram of the blood filtration device. In this example, the flow channel 3 is an artificial blood vessel. The artificial blood vessel is connected, for example, to a blood vessel of a living body. In this example, the connection device 1 is connected to the arterial port and the venous port. The film 5 is present between a blood flow channel layer and a filtrate flow channel layer. In this example, the combination of the blood flow channel layer, the film 5 and the filtrate flow channel layer is stacked in multiple levels. A filtrate is discharged from a filtrate tube.

The blood flow channel layer preferably includes a plate-shaped main body section, a groove (notch) formed in the plate-shaped main body section to form the blood flow channel and a resin layer formed on a side wall of the groove. This configuration prevents the plate-shaped main body section from contacting with blood, thereby providing a wider range of choices for the material of the plate-shaped main body section. The resin layer preferably has a shape with a gradual change in thickness, with a lowermost section and an uppermost section thicker than a center section in the height direction of the side wall of the groove. Such a shape effectively prevents clot formation in the blood flow channel. This configuration can be used for an implantable artificial kidney, a portable artificial kidney, a hemodialysis machine, a blood filtration device or an extracorporeal film oxygenator.

### EXAMPLE

A strip of PTFE sheet with a width of 2 mm and a length of 40 mm or more was cut out from a PTFE sheet.

A specified number of the strips of PTFE sheet were inserted from the circular end portion side of a connection part. In this example, four strips of PTFE sheet (0.3 × 2.0 mm) were prepared. Thus, the cross-section of the stacked strips of PTFE sheet was 1.2 × 2.0 mm, which is identical to the size of a rectangular cross-section of the connection part. When this process is performed, the diagonal length of the rectangular cross-section of the connection part is preferably designed to be equivalent to the diameter of the circular end surface of the connection part. When designed in this manner, the area of the cross-section is always constant, preventing flow velocity reduction and providing an advantage in terms of antithrombotic properties.

The tip of the strip of PTFE sheet was inserted into each inlet of stacked filters. The insertion length was approximately 5 mm.

The connection part was fixed to the stacked filter using an adhesive. When the connection part is made of PMMA, it is poor in blood compatibility, thus an inner coating was applied in advance as an antithrombotic treatment.

Drops of UV-curable adhesive 4305 (high viscosity type) were added to the portion of the strip of PTFE sheet in the gap between the connection part and the stacked filter, and then irradiated with a UV exposure machine (14 +/- 4 W/m²) for 180 s or more to be cured. This operation may be performed in multiple steps.

After the strip of PTFE sheet in the gap was completely covered, an exterior with riblets was fixed to the stacked filter.

The UV-curable adhesive 4304 is charged inside so that the inside of the exterior is filled, and then irradiated with the UV exposure machine (14 +/- 4 W/m²) for 300 s or more to be cured. Since bubbles are generated inside the resin due to its shrinkage during the curing process if the entire amount is charged at one time, it is preferable to perform the process in multiple steps. Since the filling for the inside does not contact with blood and can be made of any material, a silicone resin such as PDMS can be used. However, when the inside is filled with the silicone resin, it is preferably left at room temperature for 48 hours or more for the silicone resin to be cured.

After all curing operations were completed, the strip of PTFE sheet protruding from the connection part was pulled out using a pair of tweezers or forceps, etc.

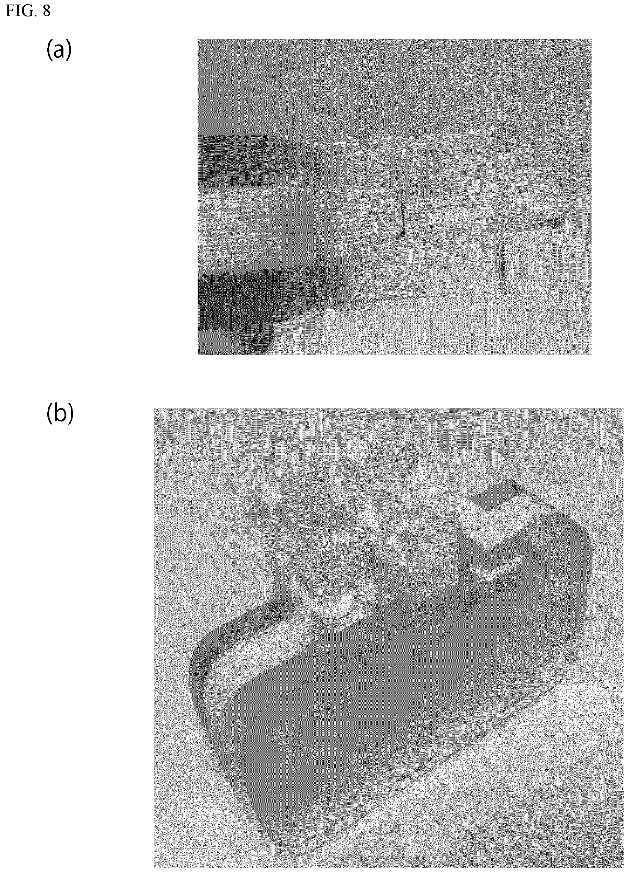

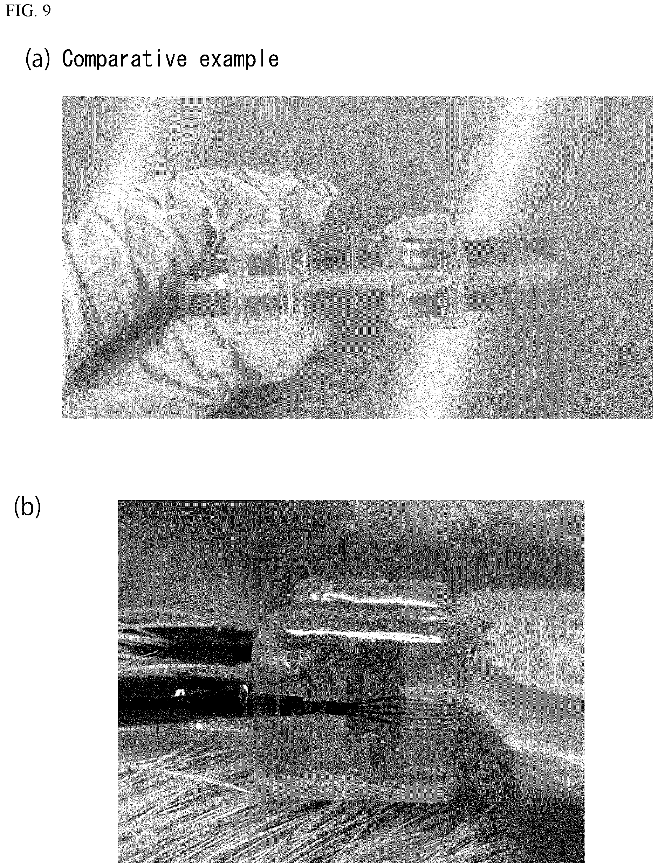

FIG. 8 is a photograph in lieu of a drawing illustrating the connection device obtained in the example. In this example, the connection device is molded with the housing as one piece. FIG. 9 is a photograph in lieu of a drawing illustrating an example of the use of the connection device. FIG. 9(a) illustrates what the connection device without the flow channel adjustment wall looks like after using it. As shown in FIG. 9(a), without the flow channel adjustment wall, a clot forms. FIG. 9(b) illustrates an example of using the connection device provided with the flow channel adjustment wall. As shown in FIG. 9(b), the flow channel adjustment wall prevented any change in the blood flow even after using the connection device for a long time, resulting in no clots.

The present invention may be used, for example, in the field of medical instruments.

### REFERENCE SIGNS LIST

- 1: Connection device
- 3: Flow channel
- 5: Film
- 7: Housing
- 9: Internal cavity
- 13: Flow channel adjustment wall
- 15: Inter-adjustment-wall flow channel
- 17: Inter-film flow channel
- 23: Virtual flow channel layer

## Claims

1. A connection device (1) having an internal cavity (9) for connecting a flow channel (3) and a housing (7) accommodating a film (5) in a state allowing fluid transport, the connection device (1) comprising
a plurality of flow channel adjustment walls (13) provided in the internal cavity (9),
wherein the film (5) is a plurality of stacked films,
an inter-adjustment-wall flow channel (15) formed by the plurality of flow channel adjustment walls (13) corresponds to an inter-film flow channel (17) formed by the plurality of stacked films (5), and
the inter-adjustment-wall flow channel (15) and the inter-film flow channel (17) are coupled when the housing (7) and the connection device (1) are connected.

2. The connection device (1) according to claim 1,
wherein the housing (7) includes a connector (21) present at an end portion of the plurality of films (5), and
each of the plurality of flow channel adjustment walls (13) is contiguous with the connector (21) when the housing (7) and the connection device (1) are connected.

3. The connection device (1) according to claim 1,
wherein the plurality of flow channel adjustment walls (13) are provided in parallel.

4. The connection device (1) according to claim 1,
wherein the internal cavity (9) includes a portion whose cross-sectional area increases in a direction from an end portion of the flow channel (3) to an end portion on a housing side.

5. A blood filtration device comprising the connection device (1) according to claim 1.

6. A method for manufacturing a connection device (1) having an internal cavity (9) for connecting a flow channel (3) and a housing (7) accommodating a plurality of stacked films (5) in a state allowing fluid transport,
wherein the internal cavity (9) is a space formed inside a main body section (11) of the connection device (1),
wherein the method comprises:
a step of installing a plurality of virtual flow channel layers (23) having a thickness corresponding to an inter-film flow channel (17) formed by the plurality of stacked films (5) inside a portion to be the main body section (11) when the connection device (1) is manufactured;
a step of forming a plurality of flow channel adjustment walls (13) between the plurality of virtual flow channel layers (23); and
a step of obtaining an inter-adjustment-wall flow channel (15) formed by the plurality of flow channel adjustment walls (13) by forming the plurality of flow channel adjustment walls (13) and then removing the virtual flow channel layer (23),
wherein the inter-adjustment-wall flow channel (15) corresponds to the inter-film flow channel (17), and
the inter-adjustment-wall flow channel (15) and the inter-film flow channel (17) are coupled when the housing (7) and the connection device (1) are connected.
